# EUROPEAN PATENT APPLICATION

(11) **EP 2 829 228 A1**
(43) Date of publication of application: **28.01.2015**
(21) Application number: 13182459.1
(22) Date of filing: 30.08.2013
(51) Int. Cl.: A61B 5/1477, G01N 27/327

(54) **Near-field-communication or RFID based electrochemical biosensor and method for an ingredient measurement using thereof**

(30) Priority: 22.07.2013 KR 20130086035
(71) Applicant: Center for Integrated Smart Sensors Foundation, Daejeon (KR)
(72) Inventor: Chong-Min, Kyung, Daejeon (KR); Cho, Hyun Tae, Daejeon (KR); Lee, Seung Ro, Daejeon (KR)
(74) Representative: Gille Hrabal

(57) **Abstract**

Disclosed herein are a near-field-communication or a Radio-Frequency Identification (RFID) based electrochemical biosensor capable of measuring an ingredient by being inter-worked with a wireless communication device such as a smartphone and a method for an ingredient measurement using thereof. The near field communication or radio-frequency identification based electrochemical biosensor, includes: a casing having a specimen introduction channel formed therein; an ingredient measurement electrode disposed in the casing and measuring a specific ingredient of a specimen; a reaction reagent portion disposed in the casing and reacting with the specimen; a specimen recognition electrode disposed in the casing and recognizing the introduction of the specimen; an antenna transmitting and receiving signals and power to and from a smart device or a tester; and a control integrated circuit (IC) chip controlling the ingredient measurement electrode, the specimen recognition electrode, and the antenna.

## Description

### CROSS REFERENCE TO RELATED APPLICATION

This application claims the benefit of Korean Patent Application No. 10-2013-0086035, filed on July. 22, 2013, entitled "Near-Field-Communication or RFID based Electrochemical Biosensor and Method for an Ingredient Measurement using thereof", which is hereby incorporated by reference in its entirety into this application.

### BACKGROUND OF THE INVENTION

### 1. Technical Field

The present invention relates to a near-field-communication or a Radio-Frequency Identification (RFID) based electrochemical biosensor and a method for an ingredient measurement using thereof, and more particularly, to an electrochemical biosensor manufactured by integrating a near-field-communication or an RFID based antenna and integrated circuit (IC) chip capable of being inter-worked with a near-field-communication or RFID enabled devices, that is, smart devices such as a smartphone, a smartpad, and the like to monitor a body ingredient such as blood, body fluid, or the like of human or animal, and a method for an ingredient measurement using thereof.

### 2. Description of the Related Art

Recently, modern people are increasingly troubled with a so-called adult disease such as diabetes, hyperlipemia, thrombosis patient, and the like caused by westernized eating habits and in a case of young women, iron deficiency anemia patients have rapidly increased due to extreme dieting. A simple method capable of checking the relative seriousness of the above-mentioned adult diseases is to measure body ingredient in blood. The body ingredient measurement may check amounts of several ingredients contained in the blood such as blood sugar, anemia, blood coagulation, and the like, such that an ordinary person may easily judge normal or abnormal conditions such as whether a numerical value of a specific ingredient is in a normal region or an abnormal region without going to a hospital.

One of the simple methods of measuring the body ingredient is to introduce the blood collected from a fingertip using an electrochemical disposable biosensor into a strip and then quantitatively analyze an output signal using an electrochemical or photometry method, wherein this method is suitable for the ordinary person having no special knowledge since a tester may display the relevant ingredient amount.

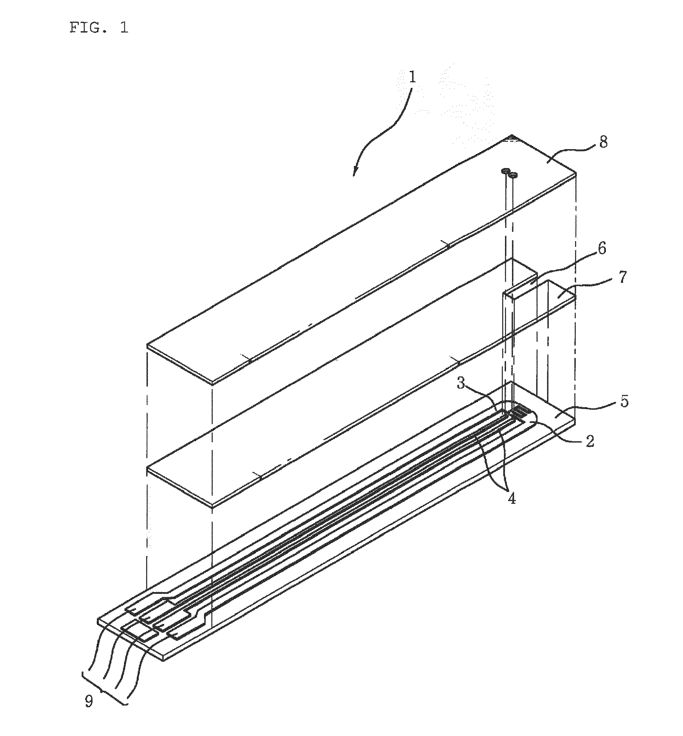

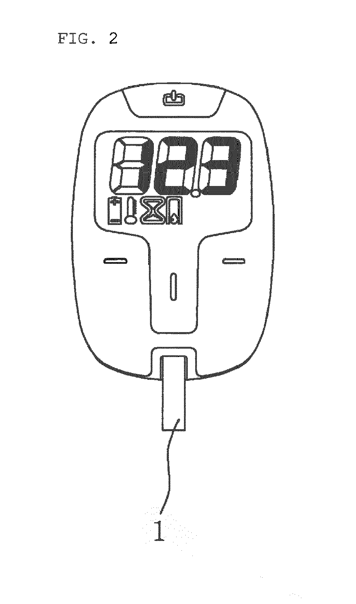

An electrochemical biosensor according to the related art is disclosed in Patent Document 1. FIG. 1 shows the electrochemical biosensor according to the related art and the electrochemical biosensor 1 according to the related art is configured of a lower plate 5 having a working electrode 2, a reference electrode 3, and a specimen recognizing electrode 4 formed thereon, an intermediate plate 7 having a specimen inserting channel 6 therein and stacked on the lower plate 5, and an upper plate 8 stacked on the intermediate plate 7, and connection terminals 9 of the working electrode 2, the reference electrode 3, and the specimen recognizing electrode 4 are inserted into the tester so as to display a specific ingredient of the blood on the tester as shown in FIG. 2.

However, the above-mentioned electrochemical biosensor according to the related art may incur economic burden because a user needs to separately purchase the tester and a problem that the user needs to carry the tester when moving such as a business trip, a travel, or the like.

### [Related Art Document]

### [Patent Document]

(Patent Document 1) KR 10-1003077 B1 (December 21, 2010)

### SUMMARY OF THE INVENTION

An object of the present invention is to provide a structure of a near field communication (NFC) or radio-frequency identification (RFID) based disposable electrochemical biosensor, in which an antenna and an integrated circuit (IC) chip are integrated, capable of diagnosing the numerical value of a body ingredient using a smart device having an NFC or RFID function embedded therein without requiring a separate tester, and a method for an ingredient measurement using thereof.

According to an exemplary embodiment of the present invention, there is provided a near field communication or radio-frequency identification based electrochemical biosensor, including: a casing having a specimen introduction channel formed therein; an ingredient measurement electrode disposed in the casing and measuring a specific ingredient of a specimen; a reaction reagent portion disposed in the casing and reacting with the specimen; a specimen recognition electrode disposed in the casing and recognizing the introduction of the specimen; an antenna transmitting and receiving signals and power to and from a smart device or a tester; and a control integrated circuit (IC) chip controlling the ingredient measurement electrode, the specimen recognition electrode, and the antenna.

According to another exemplary embodiment of the present invention, there is provided a method for an ingredient measurement using a near field communication or radio-frequency identification based electrochemical biosensor, the method including: a step of introducing a specimen introducing the specimen into the specimen introduction channel of the near field communication or radio-frequency identification based electrochemical biosensor as described above; a step of approaching the smart device or the tester to the near field communication or radio-frequency identification based electrochemical biosensor; a step of determining whether or not the specimen is introduced, supplying power to the near field communication or radio-frequency identification based electrochemical biosensor by operating an NFC function of the smart device or the tester and determining whether the specimen is sufficiently introduced by the specimen recognition electrode; a step of measuring an ingredient amount to be measured by the ingredient measurement electrode; a step of internally processing inputting the measured ingredient amount to an algorithm embedded in the processor and calculating a numerical value; a step of transmitting the calculated result to the smart device or the tester through the antenna; and a step of receiving and displaying a signal displaying the signal on a display or voice-guiding the signal by a speaker using an application embedded in the smart device or the tester when the smart device or the tester receives the signal.

### BRIEF DESCRIPTION OF THE DRAWINGS

FIG. 1 is an exploded perspective view of an electrochemical biosensor according to the related art;
FIG. 2 shows a tester coupled to the electrochemical biosensor according to the related art;
FIG. 3 is a concept view of a near field communication or radio-frequency identification based electrochemical biosensor according to an exemplary embodiment of the present invention;
FIGS. 4A and 4B are a perspective view and an exploded perspective view of the near field communication or radio-frequency identification based electrochemical biosensor according to the exemplary embodiment of the present invention;
FIG. 5 is an internal configuration view of a control integrated circuit (IC) chip in the near field communication or radio-frequency identification based electrochemical biosensor according to the exemplary embodiment of the present invention;
FIGS. 6A and 6B show an exemplary embodiment using a biosensor tester or a smart device;
FIG. 7 is a flow chart of a method for an ingredient measurement using the near field communication or radio-frequency identification based electrochemical biosensor according to the exemplary embodiment of the present invention; and
FIG. 8 is a configuration view of a medical supporting system applied with the near field communication or radio-frequency identification based electrochemical biosensor according to the exemplary embodiment of the present invention.

### DESCRIPTION OF THE PREFERRED EMBODIMENTS

Hereinafter, a near field communication or radio-frequency identification based electrochemical biosensor and a method for an ingredient measurement using thereof according to an exemplary embodiment of the present invention will be described in detail with reference to the accompanying drawings.

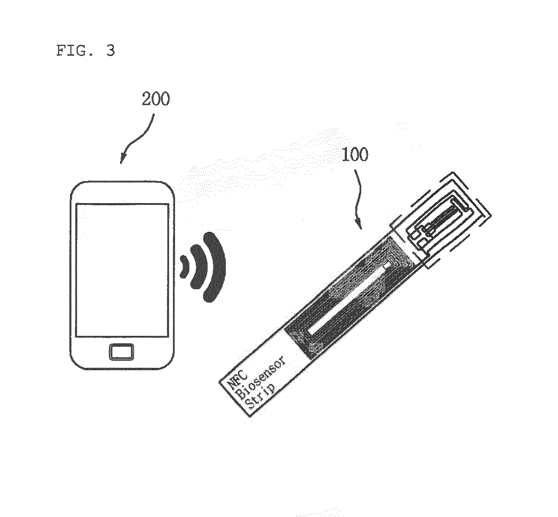

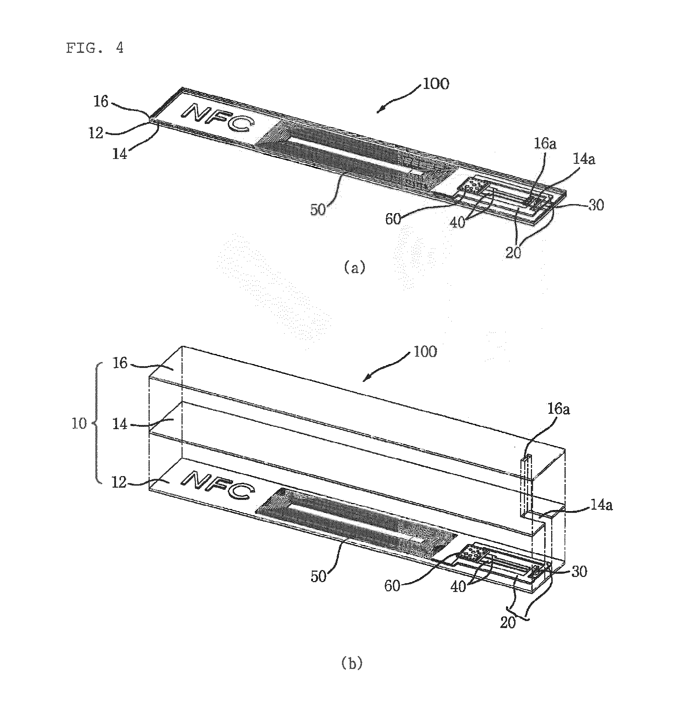

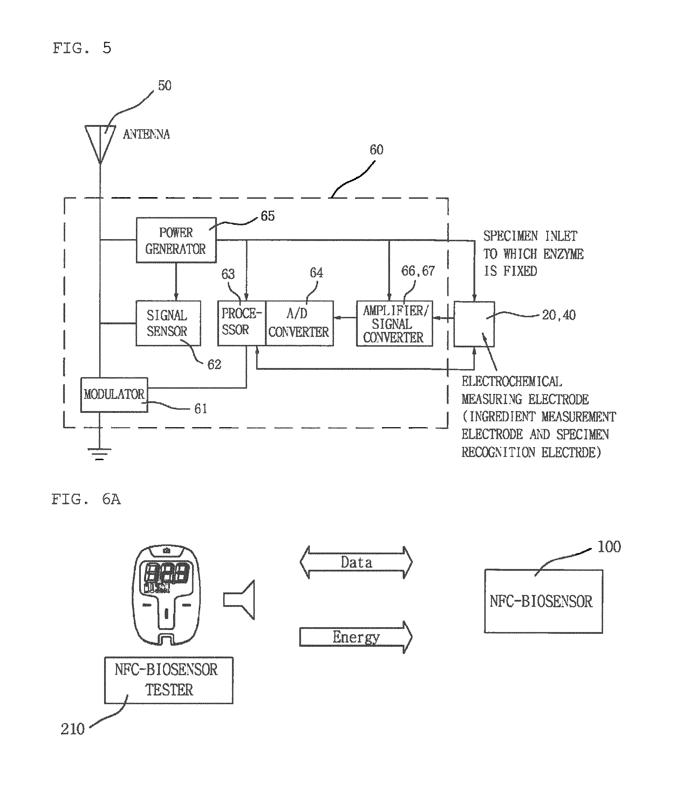

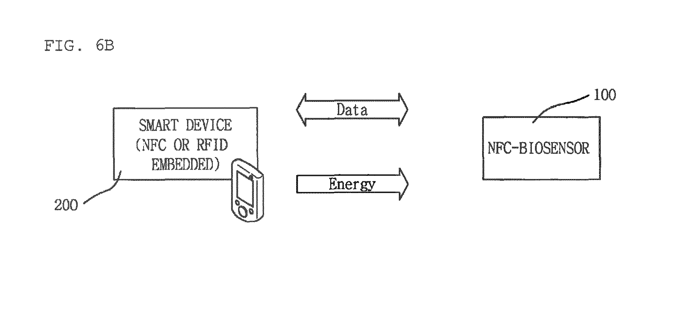

FIG. 3 is a concept view of a near field communication or radio-frequency identification based electrochemical biosensor according to an exemplary embodiment of the present invention, FIGS. 4A and 4B are a perspective view and an exploded perspective view of the near field communication or radio-frequency identification based electrochemical biosensor according to the exemplary embodiment of the present invention, FIG. 5 is an internal configuration view of a control integrated circuit (IC) chip in the near field communication or radio-frequency identification based electrochemical biosensor according to the exemplary embodiment of the present invention, and FIGS. 6A and 6B show an exemplary embodiment using a biosensor tester or a smart device.

A basic technical idea of the present invention is to display a result of a diagnosis, which is a measurement value sensed by the near field communication or radio-frequency identification based electrochemical biosensor 100 on a personal smart device 200 already dispersed to most people without separately purchasing a tester, as shown in FIG. 3.

The near field communication or radio-frequency identification based electrochemical biosensor 100 according to the exemplary embodiment of the present invention is configured to include a casing 10 having a specimen introduction channel 14a formed therein; an ingredient measurement electrode 20 disposed in the casing 10 and measuring a specific ingredient of a specimen; a reaction reagent portion 30 disposed in the casing 10 and reacting with the specimen; a specimen recognition electrode 40 disposed in the casing 10 and recognizing the introduction of the specimen; an antenna 50 transmitting and receiving signals and power to and from an external smart device 200; and a control integrated circuit (IC) chip 60 controlling the ingredient measurement electrode 20, the specimen recognition electrode 40, and the antenna 50. That is, the near field communication or radio-frequency identification based electrochemical biosensor 100 according to the exemplary embodiment of the present invention is configured of the casing 10 having the specimen introduction channel 14a formed therein, and the ingredient measurement electrode 20, the reaction reagent portion 30, the specimen recognition electrode 40, the antenna 50, and the control IC chip (hereinafter, referred to as the control IC chip and the like) formed in the casing 10, wherein the control IC chip and the like may be formed at an appropriate location in the casing 10 in consideration of convenience in manufacturing.

In consideration of the convenience in manufacturing, the casing 10 may be formed in a three-layer structure, that is, a structure in which a lower plate 12, an intermediate plate 14,and an upper plate 16 are stacked as shown in FIG. 4B. A shape of each of the upper, intermediate, and lower plates has no limitation, but may be manufactured in a strip shape for simple manufacturing, clamping, storing, packaging, and the like thereof. Hereinafter, an exemplary embodiment of the present invention having the casing 10 configured of a stacked structure of the lower plate 12, the intermediate plate 14, and the upper plate 16 will be described.

The lower plate 12 has the ingredient measurement electrode 20, the reaction reagent portion 30, the specimen recognition electrode 40, the antenna 50, and the control IC chip 60 formed thereon.

The lower plate 12 has the ingredient measurement electrode 20, the reaction reagent portion 30, the specimen recognition electrode 40, the antenna 50, and the control IC chip 60 formed on one surface thereof, and the other surface thereof is used with a component forming an external surface of the near field communication or radio-frequency identification based electrochemical biosensor 100 according to the exemplary embodiment of the present invention, for example, a polyethylene terephtalate (PET) film, a polyimide (PI) film, and the like used in a flexible printed circuit board (FPCB). Here, the reason why the PET film and the like are cited as an example of the lower plate 12 is that it is preferable to use materials capable of decreasing costs if possible, since the near field communication or radio-frequency identification based electrochemical biosensor 100 according to the exemplary embodiment of the present invention is a disposable strip which is difficult to re-use after the specimen such as blood is introduced and measured. In this context, a maximum advantage in a manufacturing process of the near field communication or radio-frequency identification based electrochemical biosensor according to the exemplary embodiment of the present invention is not a fact that each of the ingredient measurement electrode 20, the specimen recognition electrode 40, and the antenna 50 mounted on the lower plate 12 is separately formed when using the PET film, the polyimide film, and the like, but is a fact that manufacturing costs for mass-production are decreased since the ingredient measurement electrode 20, the specimen recognition electrode 40, and the antenna 50 are simultaneously formed in one process by a screen printing, gravure print, sputtering, laser patterning, wet etching, plating method, or the like. In this case, materials of the ingredient measurement electrode 20, the specimen recognition electrode 40, and the antenna 50 may be any one of gold (Au), platinum (Pt), silver (Ag), aluminum (Al), palladium (Pd), copper (Cu), copper/nickel in which the nickel is electrolessly plated to the copper, or copper/nickel/gold in which the nickel and gold are sequentially plated to the copper, and the method such as the screen printing, gravure print, sputtering, laser patterning, wet etching, plating method, or the like may be appropriately selected according to property of each metal.

The ingredient measurement electrode 20 is a component configured of a pair of electrodes adjacent to each other and capable of knowing an amount of ingredient to be measured which is contained in the specimen by measuring electrical characteristic varied by a reaction of the specimen introduced between the pair of electrodes with the reaction reagent portion 30.

The reaction reagent portion 30, which is a component allowing electrochemical characteristic of the specimen to be changed by reacting with the ingredient to be measured of the specimen, is formed so that a reaction reagent selectively reacting with a body specimen, for example, an enzyme, a particularly high molecular substance, or the like is fixed. In order for the reaction reagent portion 30 to perform a blood sugar measurement, an anemia measurement, a blood coagulation time measurement, the reaction reagent capable of reacting with the respective index ingredients needs to be appropriately selected. The reaction reagent portion 30 may be fixed on surfaces of a pair of ingredient measurement electrodes 20. The reason for this is that since the electrical characteristic of the specimen is measured by the ingredient measurement electrode 20, the fixation of the reaction reagent on the surface of the ingredient measurement electrode 20 is a structure capable of most sensitively measuring a change in the electrochemical characteristic of the specimen.

The specimen recognition electrode 40, which is a component disposed so as to be adjacent to the ingredient measurement electrode 20 and sensing whether the body specimen of a sufficient amount is introduced, may be formed at a lower end portion of the specimen introduction channel 14a between the ingredient measurement electrodes 20, as shown in FIG. 4. Alternatively, as in the related art shown in FIG. 1, the specimen recognition electrode 40 may be formed behind the ingredient measurement electrode 20, viewing from the specimen introduction channel 14a. The displacement of the specimen recognition electrode 40 shown in FIG. 4 and the displacement of the specimen recognition electrode shown in FIG. 1 are commonly the structure in which the specimen may be introduced into the specimen recognition electrode 40 only in the case in which the specimen of the sufficient amount is introduced into the ingredient measurement electrode 20. As such, the specimen recognition electrode 40 has no limitation in a shape or displacement thereof as long as it has a configuration capable of sensing whether the specimen is sufficiently introduced into the ingredient measurement electrode 20.

The antenna 50, which is a component formed on the lower plate 12 so as to transmit and receive the signal (data or control instructions) and power to and from the smart device 200 or the tester 210 which is external thereof, is typically formed in a form in which wires having a thin film or thick film form are overlapped and wound. A detailed specification (for example, a wire, a width, a pattern, or the like) of the antenna 50 is changed depending on a frequency, an arrival distance, an energy transfer amount per time, or the like of a near field communication, and since this is a design item for a person skilled in the art, the detailed description thereof will be omitted.

The control IC chip 60 is a component connected to the ingredient measurement electrode 20, the specimen recognition electrode 40, and the antenna 50 to generate power from the antenna 50 in proximity to the smart device 200 or the tester 210, performing an electrochemical signal detection from the ingredient measurement electrode 20 and the specimen recognition electrode 40 using the energy, and transmitting data of the measured ingredient to the smart device 200 or the tester 210 which is external to through the antenna 50. The control IC chip 60 is configured to include a modulator 61, a signal sensor 62, a processor 63, and an analog-to-digital (A/D) converter 64 as shown in FIG. 5, and may further include a power generator 65, an amplifier 66, or a signal converter 67, if necessary.

The modulator 61, which is a component for modulating the signal containing the measured data into a waveform suitable for transmission in order to transmit the measured data to the smart device 200 or the tester 210, modulates the measured data into signals of several bands according to the current near field communication standard and transfers the modulated signals to the antenna 50.

The signal sensor 62 is a component which senses the data or the control instructions from the smart device 200 or the tester 210 received through the antenna 50 to transfer it to the processor 63. By the modulator 61 and the signal sensor 62, communication between the near field communication or radio-frequency identification based electrochemical biosensor 100 according to the exemplary embodiment of the present invention and the smart device 200 or the tester 210 is possible.

The processor 63, which is a component processing the instructions transmitted from the smart device 200 or the tester 210, sensing a recognition of the specimen through the specimen recognition electrode 40, and dating an ingredient amount to be measured using the ingredient measurement electrode 20, is a kind of microprocessor.

The A/D converter 64, which converts an analog signal into a digital signal, converts the analog signal such as a current sensed in the ingredient measurement electrode 20 into the digital signal capable of being processed in the processor 63 and provides the converted digital signal to the processor 63.

The power generator 65 is a component generating the power necessary to drive the modulator 61, the processor 63, and the like by receiving the power from the smart device 200 or the tester 210 which is external to, when the near field communication or radio-frequency identification based electrochemical biosensor 100 according to the exemplary embodiment of the present invention is a passive type having no battery. When the battery is embedded, the power generator 65 may be omitted.

The amplifier 66, which is a component amplifying a magnitude of the signal, amplifies the signal when the signal sensed by the specimen recognition electrode 40 and the ingredient measurement electrode 20 is weak to thereby facilitate a signal processing. The amplifier 66 may be omitted when performance of the processor 63 is good or the signal sensed by the specimen recognition electrode 40 and the ingredient measurement electrode 20 has a sufficient magnitude.

The signal converter 67, which is a component converting the signal sensed by the specimen recognition electrode 40 and the ingredient measurement electrode 20 into a form suitable of processing, is a converter converting a current into a voltage, for example. Typically, since electronic components often perform a processing using a voltage value as an input, the signal converter 67 converting a current value into a voltage value is generally used. When the components in the near field communication or radio-frequency identification based electrochemical biosensor 100 according to the exemplary embodiment of the present invention are designed to process the current value, the signal converter 67 may be omitted.

The control IC chip 60 may recognize the introduction of the specimen by a change in capacitance according to a type of specimen recognition electrode 40 (a non-contacting type) and may recognize the introduction of the specimen by applying the voltage to sense a change in a flowing current amount (a contacting type). Once the introduction of the specimen is sensed by any method, the control IC chip 60 applies the voltage to the ingredient measurement electrode 20 to measure the current and measure the change thereof. A time arriving at a steady state may differ slightly depending on the reaction reagent portion 30 and a kind of ingredient reacting therewith. However, after a predetermined time is elapsed, the control IC chip 60 senses a change amount in electrical property measured by the ingredient measurement electrode 20 and then transmits it to the smart device 200 or the tester 210 which is external through the antenna 50.

The power consumed in operating the control IC chip 60 is supplied from the smart device 200 or the tester 210 which is external to through the antenna 50 or supplied from the embedded battery. In the near field communication, the control IC chip 60 is a passive type in the case in which the power is generated from the smart device 200 or the tester 210 which is external to, and is an active type in the case in which the battery capable of supplying the power is embedded. In the case of the active type, since manufacturing costs are increased due to the battery itself and an addition of a process for embedding the battery, the near field communication or radio-frequency identification based electrochemical biosensor 100 according to the exemplary embodiment of the present invention may be configured in the passive type, that is, to include the power generator 65. However, in the case in which large power is required for communication, such as a case in which the smart device 200 or the tester 210 is distant from the near field communication or radio-frequency identification based electrochemical biosensor 100 according to the exemplary embodiment of the present invention, a separate battery may be embedded in the near field communication or radio-frequency identification based electrochemical biosensor 100 according to the exemplary embodiment of the present invention.

The passive type of the control IC chip 60 including the power generator 65 will be described. In order to measure the particular ingredient using the near field communication or radio-frequency identification based electrochemical biosensor 100 according to the exemplary embodiment of the present invention, the smart device 200 or the tester 210 needs to be approached to the biosensor. (Currently, a standardized near field communication known under the name of a so-called near-field-communication (NFC) has a frequency band 13.56MHz and has a maximum operating distance of 20cm or less, generally, 10cm or less. In a case of the RFID, the frequency band or the operating distance may be changed.) When the smart device 200 or the tester 210 approaches the biosensor, an electromagnetic induction phenomenon occurs from the smart device 200 or the tester 210 to the antenna 50. In this case, the power generator 65 of the control IC chip 60 appropriately converts the power transferred by the above-mentioned electromagnetic induction phenomenon and generates a required voltage to supply it to internal components. The data regarding the measured ingredient is transferred to the smart device 200 or the tester 210 which is external to through the antenna 50 and is processed by an application or the like installed in the smart device 200 or the tester 210, such that a numerical value thereof is displayed by a display of the smart device 200 or the tester 210 or is voice-guided by a speaker of the smart device 200 or the tester 210.

The intermediate plate 14 has a plate shape and one side thereof may be provided with the specimen introduction channel 14a into which the specimen may be introduced. A material of the intermediate plate 14 may be an insulating material so as to prevent a short between the ingredient measurement electrode 20, the specimen recognition electrode 40, and the antenna 50 and needs to be a material that may be used in several frequency bands of the NFC, the RFID, and the like since the near field communication between the antenna 50 formed on the lower plate 12 and the smart device 200 or the tester 210 which is external to may need to be performed. Therefore, the material of the intermediate plate 14 may be the same PET, polyimide, and the like as the lower plate 12. The intermediate plate 14 is stacked on the lower plate 12 so as to serve to protect the ingredient measurement electrode 20, the reaction reagent portion 30, the specimen recognition electrode 40, the antenna 50, and the control IC chip 60 formed on the lower plate 12 from the outside.

The upper plate 16 has a plate shape, is stacked on an upper portion in which the lower plate 12 and the intermediate plate 14 are stacked and coupled, may have an air vent 16a formed at a location corresponding to the specimen introduction channel 14a formed in the intermediate plate 14. By sequentially stacking the lower plate 12, the intermediate plate 14, and the upper plate 16, the specimen introduction channel 14a in the intermediate plate 14 forms a capillary, such that when the specimen contacts an inlet of the specimen introduction channel 14a, the specimen is automatically introduced into the specimen introduction channel 14a by a capillary phenomenon. In this case, in the case in which the air vent 16a is formed in the upper plate 16, the amount of air that is the same as the amount of the specimen introduced through the air vent 16a is discharged, such that an internal pressure is decreased, thereby further facilitating the introduction of the specimen.

FIGS. 6A and 6B show an exemplary embodiment using a biosensor tester or a smart device. The near field communication or radio-frequency identification based electrochemical biosensor 100 according to the exemplary embodiment of the present invention, which has a structure integrating the antenna 50 therewith, may display a measured value by the tester 210 having an NFC function mounted thereon as shown in FIG. 6A or may display the measured value by the smart device 200 having an NFC/RFID function embedded therein as shown in FIG. 6B. In this case, a specific form of the antenna 50 is an NFC tag or a RFID tag. Since the basic technical ideal of the present invention is to use the smart device which is already dispersed without requiring the separate tester, the use of the NFC/RFID in the present invention may be the communication of the near field communication or radio-frequency identification based electrochemical biosensor 100 according to the exemplary embodiment of the present invention with the smart device 200 having the NFC/RFID function embedded therein as shown in FIG. 6B.

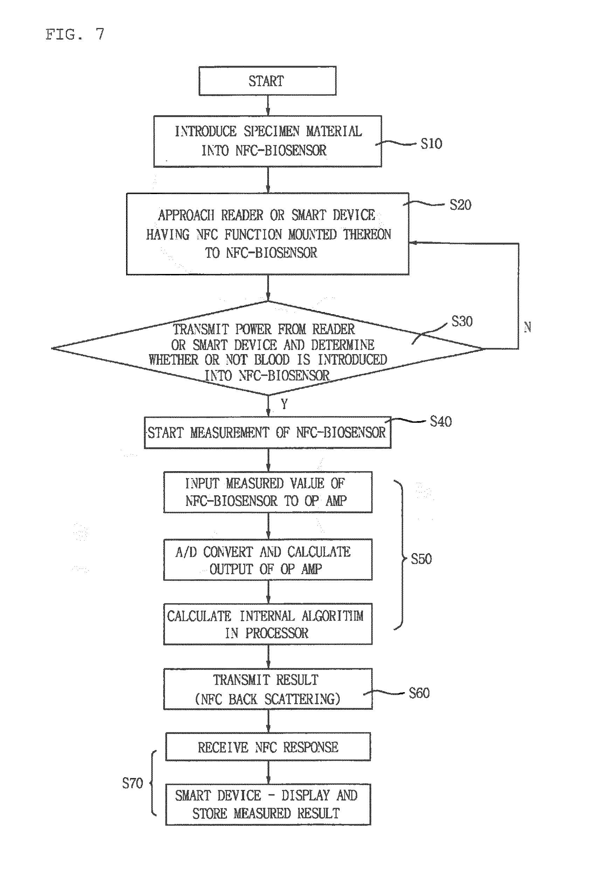

Next, a method for an ingredient measurement using the near field communication or radio-frequency identification based electrochemical biosensor according to the exemplary embodiment of the present invention will be described. FIG. 7 is a flow chart of a method for an ingredient measurement using the near field communication or radio-frequency identification based electrochemical biosensor according to the exemplary embodiment of the present invention.
① A step of introducing a specimen S10: The specimen is introduced into the specimen introduction channel 14a of the near field communication or radio-frequency identification based electrochemical biosensor 100 according to the exemplary embodiment of the present invention.
② A step of approaching S20: The smart device 200 or the tester 210 is approached to the near field communication or radio-frequency identification based electrochemical biosensor 100 according to the exemplary embodiment of the present invention.
③ A step of determining whether or not the specimen is introduced S30: the power is supplied to the near field communication or radio-frequency identification based electrochemical biosensor 100 according to the exemplary embodiment of the present invention by operating the NFC function of the smart device 200 or the tester 210, and whether the specimen is sufficiently introduced is determined by the specimen recognition electrode 40. Once it is determined that the specimen is sufficiently introduced, proceeds to a next step, and in the case in which the specimen is insufficiently introduced, whether or not the specimen is introduced is continuously measured while maintaining the step of approaching S20.
④ A step of measuring S40: An ingredient amount to be measured is measured by the ingredient measurement electrode 20.
⑤ A step of internally processing S50: The measured ingredient amount is amplified and analog-to-digital converted, if necessary and is then input to an algorithm embedded in the processor 63, such that a numerical value is calculated.
⑥ A step of transmitting S60: The calculated result is transmitted to the smart device 200 or the tester 210 which is external to through the antenna 50.
⑦ A step of receiving and displaying S70: When the smart device 200 or the tester 210 receives a signal, the signal is displayed on the display or is voice-guided by the speaker using the application and the like embedded in the smart device 200 or the tester 210.

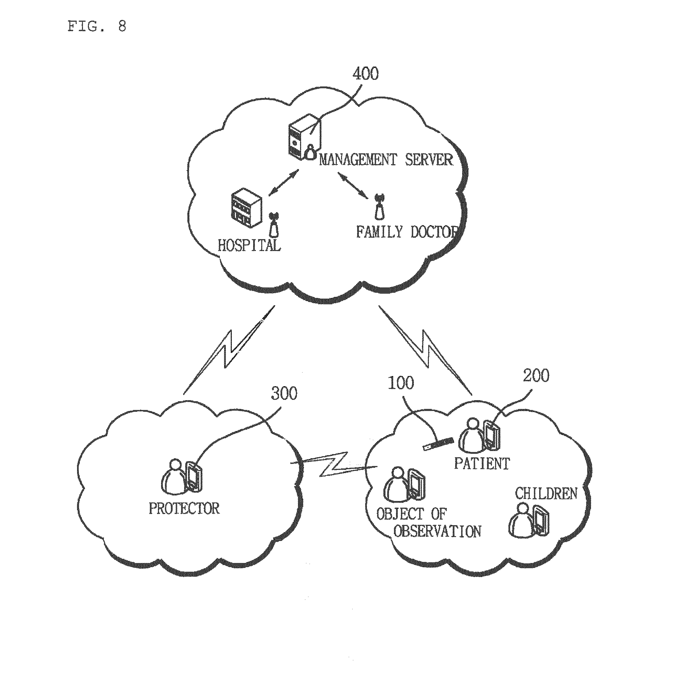

Next, a medical supporting system will be described as an application example using the near field communication or radio-frequency identification based electrochemical biosensor according to the exemplary embodiment of the present invention. FIG. 8 is a configuration view of a medical supporting system applied with the near field communication or radio-frequency identification based electrochemical biosensor according to the exemplary embodiment of the present invention.

The medical supporting system applied with the near field communication or radio-frequency identification based electrochemical biosensor according to the exemplary embodiment of the present invention is configured to include the near field communication or radio-frequency identification based electrochemical biosensor 100 according to the exemplary embodiment of the present invention, the smart device 200 or the tester 210 of a user, a smart device 300 of a protector, and a management server 400. Hereinafter, a case in which blood of the user is used as the specimen will be described.

Since the near field communication or radio-frequency identification based electrochemical biosensor 100 according to the exemplary embodiment of the present invention and the smart device 200 or the tester 210 of the user are described above, an overlapped description thereof will be omitted.

The smart device 300 of the protector may receive information such as a blood sugar amount of the user, and the like from the smart device 200 of the user or the management server 400 to observe a health status of the user. Therefore, for example, in the case in which it is expected that the user will go into a hypoglycemic state, the protector keeps in touch with the user to direct the user to ingest sugar or the protector may take care of the user before the user loses consciousness, such that it prevents the user from danger.

The management server 400 may store the blood sugar amounts of the user repeatedly measured so as to allow the user, the protector or a doctor to monitor a history of the health status of the user. In a case of the doctor, the doctor may accurately diagnose a past or current state of the user by checking the history stored in the management server 400 without repeatedly asking the same questions to the user or the protector. In addition, the management server 400 may periodically transmits a message requiring a blood sugar measurement to the smart device 200 or tester 210 of the user, such that it may prevent the user from mistakenly missing a time for measuring the blood sugar.

According to the exemplary embodiment of the present invention, since the near field communication or radio-frequency identification based electrochemical biosensor does not require the need to separately purchase the tester but require to separately purchase the strip one at a time, it may be excellent in terms of economical efficiency. In addition, since the portable smart devices, which are individual necessities are used, the field diagnosis for the blood sugar, the anemia, the blood coagulation time, and the like may be possible even when not mistakenly carrying the tester at the time of the travel, or the like.

## Claims

1. A near field communication or radio-frequency identification based electrochemical biosensor, comprising:
a casing having a specimen introduction channel formed therein;
an ingredient measurement electrode disposed in the casing and measuring a specific ingredient of a specimen;
a reaction reagent portion disposed in the casing and reacting with the specimen;
a specimen recognition electrode disposed in the casing and recognizing the introduction of the specimen;
an antenna transmitting and receiving signals and power to and from a smart device or a tester; and
a control integrated circuit (IC) chip controlling the ingredient measurement electrode, the specimen recognition electrode, and the antenna.

2. The near field communication or radio-frequency identification based electrochemical biosensor of claim 1, wherein the casing is formed in a structure in which a lower plate, an intermediate plate, and an upper plate are stacked,
the lower plate has the ingredient measurement electrode, the reaction reagent portion, the specimen recognition electrode, the antenna, and the control IC chip formed thereon, and
the intermediate plate has a specimen introduction channel formed therein.

3. The near field communication or radio-frequency identification based electrochemical biosensor of claim 1, wherein the reaction reagent portion is formed so that a reaction reagent selectively reacting with a body specimen is fixed onto a pair of electrode surfaces of the ingredient measurement electrode.

4. The near field communication or radio-frequency identification based electrochemical biosensor of claim 1, wherein the control IC chip is connected to the ingredient measurement electrode, the specimen recognition electrode, and the antenna to receive an electrical signal from the ingredient measurement electrode and the specimen recognition electrode and transmit data of the measured ingredient to the smart device or the tester through the antenna.

5. The near field communication or radio-frequency identification based electrochemical biosensor of claim 1, wherein the control IC chip is configured to include:
a modulator modulating the signal containing the measured data into a waveform suitable for transmission;
a signal sensor sensing data or control instructions from the smart device or the tester received through the antenna;
a processor processing the instructions transmitted from the smart device or the tester, sensing a recognition of the specimen through the specimen recognition electrode, and dating an ingredient amount to be measured using the ingredient measurement electrode; and
an analog-to-digital converter converting an analog signal into a digital signal.

6. The near field communication or radio-frequency identification based electrochemical biosensor of claim 5, wherein the control IC chip is configured to further include any one or more of a power generator generating necessary power by receiving the power from the smart device or the tester, an amplifier amplifying a magnitude of a signal, or a signal converter 67 converting a current into a voltage.

7. The near field communication or radio-frequency identification based electrochemical biosensor of claim 1, wherein the control IC chip recognizes the introduction of the specimen by a non-contact type recognizing the introduction of the specimen by measuring capacitance or a contact type recognizing the introduction of the specimen by applying a voltage to sense a change in a flowing current amount.

8. The near field communication or radio-frequency identification based electrochemical biosensor of claim 1, wherein the antenna is an NFC tag or a RFID tag.

9. The near field communication or radio-frequency identification based electrochemical biosensor of claim 1, wherein a material of the ingredient measurement electrode, the specimen recognition electrode, and the antenna is any one of gold, platinum, silver, aluminum, palladium, copper, copper/nickel in which the nickel is electrolessly plated to the copper, or copper/nickel/gold in which the nickel and gold are sequentially plated to the copper.

10. A method for an ingredient measurement using a near field communication or radio-frequency identification based electrochemical biosensor, the method comprising:
a step of introducing a specimen introducing the specimen into the specimen introduction channel of the near field communication or radio-frequency identification based electrochemical biosensor of any one of claims 1 to 9;
a step of approaching the smart device or the tester to the near field communication or radio-frequency identification based electrochemical biosensor;
a step of determining whether or not the specimen is introduced, supplying power to the near field communication or radio-frequency identification based electrochemical biosensor by operating an NFC function of the smart device or the tester and determining whether the specimen is sufficiently introduced by the specimen recognition electrode;
a step of measuring an ingredient amount to be measured by the ingredient measurement electrode;
a step of internally processing inputting the measured ingredient amount to an algorithm embedded in the processor and calculating a numerical value;
a step of transmitting the calculated result to the smart device or the tester through the antenna; and
a step of receiving and displaying a signal displaying the signal on a display or voice-guiding the signal by a speaker using an application embedded in the smart device or the tester when the smart device or the tester receives the signal.

11. The method of claim 10, wherein in the step of determining of whether or not the specimen is introduced, when it is determined that the specimen is introduced, the step of measuring is performed, and when it is determined that the specimen is not introduced, whether or not the specimen is introduced is continuously measured while maintaining the step of approaching.

12. The method of claim 10, wherein in the step of determining of whether or not the specimen is introduced, the introduction of the specimen is recognized by a non-contact type recognizing the introduction of the specimen by a change in capacitance or a contact type recognizing the introduction of the specimen by applying a voltage to sense a change in a flowing current amount.
